# EUROPÄISCHE PATENTSCHRIFT

(11) **EP 3 569 171 B1**
(45) Veröffentlichungstag und Bekanntmachung des Hinweises auf die Patenterteilung: **13.03.2024**
(21) Anmeldenummer: 18172105.1
(22) Anmeldetag: 14.05.2018
(51) Int. Cl.: A61B 18/04, A61B 18/12, A61B 18/14

(54) **VORRICHTUNG UND VERFAHREN ZUM VORGEBEN VON BETRIEBSPARAMETERN ZUR ERZEUGUNG EINES PLASMAS IN WÄSSRIGER UMGEBUNG**
DEVICE AND METHOD FOR SETTING PARAMETERS FOR CREATING PLASMA IN AN AQUEOUS ENVIRONMENT
DISPOSITIF ET PROCÉDÉ DE SPECIFICATION DES PARAMETRES DE FONCTIONNEMENT POUR LA PRODUCTION D'UN PLASMA DANS UN MILIEU AQUEUX

(43) Veröffentlichungstag der Anmeldung: 20.11.2019
(73) Patentinhaber: Erbe Elektromedizin GmbH, 72072 Tübingen (DE)
(72) Erfinder: Sauter, Michael, 72461 Albstadt (DE); Schulz, Florian, 72108 Rottenburg (DE)
(74) Vertreter: Rüger Abel Patentanwälte PartGmbB

(56) Entgegenhaltungen:
- EP-A1- 2 108 325
- DE-A1-102017 110 473
- US-A1- 2004 049 180
- US-A1- 2005 177 150
- US-A1- 2014 309 632
- US-A1- 2016 302 843
- US-A1- 2017 202 616

## Beschreibung

Die Erfindung betrifft ein Gerät zur Speisung eines medizinischen Instruments zur Behandlung von biologischem Gewebe durch Einwirkung eines Plasmas sowie ein Verfahren zum Vorgeben von Betriebsparametern zum Zünden und Aufrechterhalten eines Plasmas in wässriger Umgebung.

Medizinische Instrumente, die mittels eines von einer Elektrode ausgehenden Plasmas auf biologisches Gewebe einwirken, sind grundsätzlich bekannt und in Gebrauch.

Dazu offenbart z.B. die EP 2 514 380 A1 ein Elektroskalpell, das zum Schneiden von biologischem Gewebe dient. Dazu wird an einer spatelförmigen Elektrode ein als Funke bezeichnetes Plasma erzeugt, das auf das Gewebe schneidende Wirkung ausübt. Das Zünden des Plasmas erfolgt in wässrig-feuchter Umgebung, indem zunächst an der Elektrode befindliches Wasser verdampft und in der Dampfschicht dann der Funke gezündet wird. Um die erforderliche Dampfschicht zu erzeugen und damit die Trocknung der Elektrode möglichst schnell zu erreichen, wird während dieser Aufheizphase mit einem größeren Puls/Pause-Verhältnis der pulsbreitenmodulierten, an der Elektrode anstehenden HF-Spannung oder sogar mit unterbrechungsfreier HF-Spannung gearbeitet, während bei dem nachfolgenden Schneidvorgang mit niedrigerem Puls-/Pause-Verhältnis gearbeitet wird.

Die DE 10 2014 217 362 A1 befasst sich mit der Plasma-Vaporisation mittels eines als Resektoskop bezeichneten Instruments, das von einem Hochfrequenz-Chirurgiegerät gespeist wird. Bei dem damit durchzuführenden chirurgischen Eingriff befindet sich das Instrument in der Regel in einer Kochsalzlösung, die als Spülfluid dient. Durch Anlegen der Hochfrequenzspannung an die Vaporisationselektrode des Instruments wird ein Plasma gezündet. Dazu wird zunächst eine Dampfschicht um die Vaporisationselektrode erzeugt, wofür hohe Leistungen und Ströme erforderlich sind, die das Spülfluid und damit das umgebene biologische Gewebe stark erwärmen können. Zur Vermeidung einer zu starken Erwärmung der Spülflüssigkeit und gleichzeitig zur Erleichterung der Plasmazündung ist eine Sperrvorrichtung vorgesehen, mit der der Spülfluiddurchfluss in einer Spülfluidleitung gesperrt oder reduziert werden kann. Damit soll nicht nur die Plasmazündung erleichtert sondern auch der Energieeintrag des Zündvorgangs vermindert werden.

Die US 2005/0177150 A1 offenbart einen Generator zur Speisung eines Instruments mit einem zur medizinischen Behandlung eines Patienten geeigneten Strom. Das Instrument dient der Gewebekoagulation und dem Gewebeschnitt und es weist dazu als Elektrode eine Drahtschlinge auf. Es ist eine Ausführungsform des Generators angegeben, bei der dieser den elektrischen Widerstand zwischen der aktiven Elektrode und der Neutralelektrode erfasst und in Abhängigkeit vom Erreichen einer Schwelle aufeinanderfolgende Behandlungsphasen weiterschaltet.

Aus der EP 2 108 325 A1 ist ein Generator mit Instrumentenerkennung bekannt, der anhand eines dem Instrument individuell zugeordneten Codes Einstellungen für Widerstandsbereiche für Leerlauf und Gewebekontakt sowie für Leistungsniveaus vornimmt. Während des Betriebs wird der Widerstand an der Elektrode gemessen und die Energieabgabe des Generators entsprechend gesteuert.

US2016/302843 A1 offenbart einen weiteren Stand der Technik.

Zur Durchführung von Geweberesektionen können dem Behandler unterschiedliche Instrumente mit verschiedenen Elektrodenformen zur Verfügung gestellt werden, die unterschiedliche Zündwilligkeiten und unterschiedliche Plasmahaltefähigkeiten aufweisen. Außerdem sind beim Zünden des Plasmas gelegentlich neurosmuskuläre Stimulationen zu verzeichnen, die den Behandler irritieren oder den Behandlungsprozess stören können. Ebenso störend können Plasmainstabilitäten oder übermäßiges Funkenspiel beim Zünden wirken.

Es ist Aufgabe der Erfindung, eine Vorrichtung und ein Verfahren anzugeben, die bzw. das wenigstens hinsichtlich eines der oben genannten Aspekte Abhilfe schafft.

Diese Aufgabe wird mit der Vorrichtung bzw. dem Gerät nach Anspruch 1, wie auch mit dem Verfahren nach Anspruch 13 gelöst:

Zu der Vorrichtung gehört ein Gerät, das einen Generator zur Abgabe einer hochfrequenten Wechselspannung sowie ein Netzteil aufweist, das mit dem Generator verbunden ist, um diesen mit Betriebsspannung zu versorgen. Der Generator ist mit einem Ausgang verbunden, an dem ein Instrument anschließbar ist. Weiter umfasst das Gerät eine Messeinrichtung zur Bestimmung des elektrischen Widerstands des an den Ausgang angeschlossenen Instruments. Dabei wird als Widerstand des Instruments jede Größe verstanden, die als Quotient aus der an dem Ausgang anliegenden Spannung und dem durch das Instrument fließenden Strom verstanden werden kann, wenn zur Messung Gleichspannung genutzt wird. Wird zur Messung Wechselspannung genutzt, ist der Widerstand der Realteil der Impedanz, die der Quotienten aus Spannung und Strom ist. Anstelle des Widerstands kann bei der vorliegenden Erfindung auch immer die Impedanz, der Scheinwiderstand, der Blindwiderstand oder der Wirkwiderstand genutzt werden. Der Widerstand des Instruments wird somit im Wesentlichen von dem Überganswiderstand von der Elektrode zur wässrigen Umgebung sowie dem Widerstand der wässrigen Umgebung selbst bis zur Rückführungselektrode bestimmt.

Das Gerät enthält außerdem eine Steuereinrichtung, die dazu eingerichtet ist, die Betriebsspannung und/oder die an das Instrument abgebbare Leistung und/oder den an das Instrument lieferbaren Maximalstrom entsprechend des gemessenen Widerstands wenigstens für eine Zeitspanne vorzugeben und dabei das Netzteil und/oder den Generator entsprechend zu steuern. Die genannte Zeitspanne kann insbesondere eine vorgegebene variable oder konstante Zeitspanne sein, die als Zündversuchszeit zum Aufbau eines Plasmas vorgesehen ist. Die Messeinrichtung kann Bestandteil der Steuereinrichtung sein.

Die Steuereinrichtung kann außerdem dazu eingerichtet sein, nach Ablauf der Zündversuchszeit für die Betriebsspannung und/oder die an das Instrument abgebbare Leistung und/oder den an das Instrument lieferbaren Maximalstrom anderweitige Vorgaben zu machen, die von den Vorgaben der Zündversuchszeit abweichen.

Mit der spezifischen Vorgabe der Spannung (d.h. des Werts) der hochfrequenten Wechselspannung und/oder der von dem Netzteil bereitgestellten Betriebsspannung für die Zündversuchszeit und/oder der Begrenzung der Leistung während der Zündversuchszeit und/oder der Begrenzung des von dem Netzteil und/oder dem Hochfrequenzgenerator maximal lieferbaren Stroms, jeweils anhand des gemessenen minimalen Widerstands an der in die Flüssigkeit eingetauchten, aber nicht gezündeten Elektrode, kann erfindungsgemäß eine Anpassung des Zündvorgangs an unterschiedlichen Instrumenten mit unterschiedlichen Elektrodenformen erreicht werden. Dabei hat sich herausgestellt, dass ein reproduzierbarer Zusammenhang zwischen dem minimalen, an dem Instrument zu messenden Widerstand und denjenigen Werten für die Betriebsspannung, die maximale Leistung und/oder den maximalen Strom besteht, bei denen eine sichere und stabile Plasmazündung bei minimalem Funkenspiel und minimalen oder zumindest geringen neuromuskulären Stimulationen erreicht werden kann.

Insbesondere hat sich herausgestellt, dass durch die für die Zündzeit spezifische Festlegung der Betriebsspannung, insbesondere die Festlegung einer Betriebsspannung, die höher (z.B. Sollwert max. 550Vp (Vp steht für die Spitzenspannung)) als die zum späteren Aufrechterhalten des Plasmas zur Verfügung stehenden Betriebsspannung (z.B. höchstens 460Vp) ist, neuromuskuläre Stimulationen minimierbar sind und sich eine geringere Zündfunkenintensität im Übergang vom ungezündeten Zustand zur Ausbildung des Plasmas zeigt. Gleichfalls wirkt die Begrenzung des bei nasser Elektrode ohne Funken fließenden maximalen Stroms sowie der Leistung für den Zündbetrieb, insbesondere die instrumentenspezifische Begrenzung des maximalen Stroms und der maximalen Leistung, mildernd bis beseitigend hinsichtlich der neuromuskulären Stimulationen.

Im genannten Sinne kann eine Strombegrenzung und/oder eine Leistungsbegrenzung, d.h. die Begrenzung des maximalen Stroms am Netzteil oder an dem Hochfrequenzgenerator zur Verbesserung des Zündvorgangs und zur Plasmastabilisierung beitragen, indem die Ausbildung übergroßer Dampfblasen und das Ablösen derselben von der Elektrode des Instruments vermieden wird.

Zur Steuerung der Betriebsspannung kann das Netzteil einen Spannungsregeleingang aufweisen, der mit der Steuereinrichtung verbunden ist. Über diesen Spannungsregeleingang wird die von dem Netzteil abzugebende Betriebsspannung vorgegeben. Jedoch ist damit zu rechnen, dass trotz der im Netzteil typischerweise vorhandenen Regelschleife zur Einregulierung der gewünschten Betriebsspannung an dem Ausgang des Netzteils Betriebsspannungsschwankungen auftreten können und zwar insbesondere Spannungsänderungen infolge schneller Laständerungen. Laständerungen erzeugen eine Erhöhung der vom Netzteil abgegebenen Betriebsspannung aufgrund der im Ausgangsfilter des Netzteils gespeicherten Energie. Außerdem können Spannungsänderungen von der endlichen Reaktionszeit der im Netzteil vorhandenen Spannungsregelschleife herrühren. Mit einer Begrenzung der Betriebsspannung während des Zündbetriebs auf einen verminderten Wert kann erreicht werden, dass ein infolge des Zündens des Plasmas sprungartiger Widerstandsanstieg an der Elektrode und somit ein sprungartiger Stromrückgang nicht zu einer übermäßigen kurzzeitigen Erhöhung der Betriebsspannung führt. Dadurch kann in der Übergangsphase (das heißt im Übergang vom Quasi-Kurzschluss bei nasser Elektrode zur Dampf- und Plasmabildung) eine Überhöhung des Leistungseintrags vermieden werden. Gleichzeitig wird die Blasenbildung reduziert und so das Plasma stabilisiert. Somit kann übermäßiges Funkenspiel ebenso wie unerwünschte neuromuskuläre Stimulation vermieden oder reduziert werden.

Alternativ oder zusätzlich weisen das Netzteil und/oder der Generator einen Strombegrenzungseingang auf, der mit der Steuereinrichtung verbunden ist. Damit wird der maximale von dem Instrument abgebbare Strom und somit der Strom bei nicht gezündetem Plasma begrenzt, der von dem Instrument in die umgebende wässrige Flüssigkeit fließt. Dies begrenzt einerseits den Leistungseintrag in die Flüssigkeit und das Gewebe und stabilisiert auch den Plasmazündvorgang.

Zusätzlich oder alternativ können das Netzteil und/oder der Generator einen Leistungsbegrenzungseingang aufweisen, der mit der Steuereinrichtung verbunden ist. Das an dem Leistungsbegrenzungseingang gegebene Signal gibt die von dem Generator maximal abgebbare Leistung vor. Die so bewirkbare Leistungsbegrenzung vermeidet eine Gewebeschädigung, eine Instrumentenschädigung und ein übermäßiges Funkenspiel und stabilisiert den Zündvorgang.

Die Steuereinrichtung kann eine Betriebsstatuserkennungseinrichtung umfassen, die dazu eingerichtet ist, den Beginn eines Zündversuchs zu erkennen. Wird ein solcher erkannt, kann die Steuereinrichtung während des Zündversuchs einen Sollwert für die Betriebsspannung vorgeben, der höher ist als der Sollwert während des Betriebs mit stabilem Plasma im Anschluss an den Zündversuch.

Die Betriebsstatuserkennungseinrichtung kann auch dazu eingerichtet sein, zu Ende eines Zündversuchs zu unterscheiden, ob ein stabiles Plasma gezündet worden ist oder ob kein Plasma vorliegt. Auf Basis dieser Information kann der weitere Betrieb des Geräts gesteuert werden. Wurde ein stabiler Plasmaaufbau erkannt, werden weitere Zündversuche unterbunden und Betriebsspannung und/oder maximale Leistung und/oder maximaler Strom werden auf für den Betrieb mit stabilem Plasma geeignete Werte gesetzt. Wurde hingegen kein stabiler Plasmaaufbau erkannt, wird der Zündversuch wiederholt. Dazu kann vorgegeben sein, dass die Steuereinrichtung nach einem fehlgeschlagenen Zündversuch zunächst eine Wartephase einhält bevor ein erneuter Zündversuch gestartet wird. Damit kann verhindert werden, dass ein zu hoher Energieeintrag in die wässrige Flüssigkeit und/oder das biologische Gewebe erfolgt.

Die Steuereinrichtung kann weiter dazu eingerichtet sein, die an das Instrument gelieferte elektrische Arbeit zu erfassen, die als Integral der elektrischen Leistung über eine Zeitspanne zu verstehen ist. Beispielsweise kann zur Erfassung der elektrischen Arbeit eine vorgegebene Zeitspanne, wie zum Beispiel eine Sekunde dienen, innerhalb derer die elektrische Leistung aufintegriert bzw. in kleinen Schritten aufsummiert wird. Zum Beispiel kann dies in Sekundenintervallen oder auch in abweichenden Zeitintervallen erfolgen. Bei einer vorteilhaften Ausführungsform kann dabei vorgesehen sein, dass die Steuereinrichtung die Wartephase zwischen zwei Zündversuchen verkürzt oder ohne Wartephase arbeitet, solange die für ein gegebenes Zeitintervall maximal zulässige elektrische Arbeit noch nicht erreicht ist. Auf diese Weise wird ein zu großer Energieeintrag ebenso vermieden, wie eine zu lange, den Behandler irritierende oder störende Wartezeit zwischen aufeinanderfolgenden Zündversuchen.

Die Steuereinrichtung kann außerdem dazu eingerichtet sein, eine Annäherung der Elektrode des Instruments an das das Lumen begrenzende biologische Gewebe zu erfassen. Dazu kann der elektrische Widerstand zwischen der Elektrode und der am Instrument vorgesehenen oder alternativ separat am Patienten angebrachten Neutralelektrode gemessen werden, der sich bei Annäherung der Elektrode an das Gewebe verändert. Wird eine solche Annäherung erfasst, kann die Steuereinrichtung einen Zündversuch starten. Bei einer vorteilhaften Ausführungsform kann dazu vorgesehen sein, dass die Steuereinrichtung, wenn sie sich in einer Wartephase zwischen zwei Zündversuchen befindet, diese verkürzt oder ohne Wartephase arbeitet, solange die für ein gegebenes Zeitintervall maximal zulässige elektrische Arbeit noch nicht erreicht ist.

Im weiteren kann das erfindungsgemäße Gerät dazu eingerichtet sein, nicht nur die Betriebsspannung und/oder die Leistung und/oder den Maximalstrom für die Zündphase, sondern auch die Betriebsspannung und/oder die Leistung und/oder den Maximalstrom für die nachfolgende Betriebsphase anhand des gemessenen Widerstands und somit instrumentenspezifisch anzupassen.

Durch einzelne, mehrere oder die Summe aller vorgenannten Merkmale lassen sich Geräte schaffen, die verschiedene angeschlossene Instrumente automatisch so speisen, dass niedrige oder keine neuromuskulären Stimulationen auftreten, zugleich gute Plasmazündeigenschaften bei geringem Funkenspiel sowie auch eine stabile Plasmahaltung erreicht werden.

Insbesondere zur Steuerung Leistungsabgabe eines Geräts zur Speisung eines medizinischen Instruments zur Behandlung von biologischem Gewebe durch Einwirkung eines Plasmas, kann das Gerät aufweisen:
- einen Generator zur Abgabe einer hochfrequenten Wechselspannung (HF-Spannung) und zur Lieferung eines hochfrequenten Stroms (HF-Strom), wobei der Generator mit einem Ausgang verbunden ist, an den das Instrument anschließbar ist,
- ein Netzteil, das mit dem Generator verbunden ist, um diesen mit einer Betriebsspannung zu versorgen,
- eine Steuereinheit zur Steuerung des Generators und/oder des Netzteils, die eine Messeinrichtung zur Bestimmung des an den Ausgang gelieferten HF-Stroms aufweist,
wobei die Steuereinrichtung dazu eingerichtet ist, wenigstens für eine Zeitspanne den an den Ausgang lieferbaren maximalen HF-Strom und/oder die an den Ausgang abgebbare Leistung vorzugeben und das Netzteil und/oder den Generator entsprechend zu steuern.

Die genannte Zeitspanne kann insbesondere diejenige Zeit enthalten, in denen sich an einer noch nassen, mit einem Flüssigkeitskörper in Berührung stehenden Elektrode eine Dampfblase bildet, wobei sich in der Dampfblase ein elektrisch leitendes Plasma bildet. Solange die Elektrode großflächig den Flüssigkeitskörper berührt, begrenzt die Steuereinrichtung den an den Ausgang gelieferten HF-Strom, d.h. der Generator arbeitet in Strombegrenzung und die anliegende Spannung ist gering. Bildet sich eine Dampfblase, springt der an dem Ausgang wirksame elektrische Widerstand um mindestens eine, typischerweise mehrere Größenordnungen nach oben, womit ein schneller Abfall des HF-Stroms, ein schneller Anstieg der HF-Spannung und auch ein Anstieg der von dem Generator gelieferten Leistung verbunden ist.

Bei dem oben genannten Gerät kann vorgesehen sein, dass die Steuereinrichtung den Abfall des HF-Stroms und/oder den Anstieg der HF-Spannung erfasst und auf dieser Basis den Maximalwert für den HF-Strom und/oder die Maximalleistung neu festlegt, insbesondere begrenzt. Die Strommessung und/oder Spannungsmessung und die Festlegung der Maximalleistung kann periodisch in kurzen Zeitabständen, z.B. von 10 µs oder 100ps erfolgen.

Zur Steuerung der Betriebsspannung, zur Begrenzung des HF-Stroms oder der Leistung können das Netzteil und/oder der Generator einen entsprechenden Regeleingang aufweisen, der mit der Steuereinrichtung verbunden ist. Über den Regeleingang kann die von dem Netzteil abzugebende Betriebsspannung und/oder der von dem Netzteil maximal abgebbare Strom und/oder die von dem Netzteil maximal abgebbare Leistung vorgegeben werden. Ist der Regeleingang an dem Generator vorgesehen, kann dadurch der maximale von dem Generator abgebbare HF-Strom und/oder die von dem Generator maximal abgebbare Leistung und/oder die von dem Generator maximal abgebbare HF-Spannung vorgegeben werden.

Zur Erfassung eines schnellen Stromabfalls, der als Indikator für die Ausbildung einer Dampfblase dient, kann das Verhältnis des zu einem aktuellen Zeitpunkt erfassten HF-Stroms zu dem HF-Strom zu einem früheren Zeitpunkt dienen. Der frühere Zeitpunkt kann bei periodischer Strommessung eine oder zwei Messungen früher liegen. Ist das Verhältnis nahe 1, liegt keine Dampfblasenbildung vor. Ist das Verhältnis wesentlich kleiner als 1, liegt eine Dampfblasenbildung vor. Dies kann als Signal zur Reduzierung bzw. Begrenzung der Leistung des Generators genommen werden. Diese Maßnahme stabilisiert die Plasmabildung, indem ein vorzeitiges Ablösen der Dampfblase von der Elektrode verhindert wird. Auch wird der entstehende Funken abgeschwächt.

Weitere Einzelheiten vorteilhafter Ausführungsformen der Erfindung sind Gegenstand der Beschreibung, der Zeichnung oder von Ansprüchen. Es zeigen:
Figur 1 das erfindungsgemäße Gerät, ein angeschlossenes Instrument und ein zu behandelndes biologisches Objekt, in schematisierter Darstellung,
Figur 2 das Instrument in einem Hohlorgan, umgeben von wässriger Flüssigkeit, in schematisierter Darstellung,
Figur 3 verschiedene Instrumente, die an dem Gerät nach Figur 1 einsetzbar sind,
Figur 4 Diagramme für verschiedene Einstellungen verschiedener Instrumente für das Zünden eines Plasmas,
Figur 5 das Verhalten von Strom und Spannung an einem Instrument beim Zünden eines Plasmas,
Figur 6 ein Diagramm verschiedener Zündszenarien und
Figur 7 ein Diagramm zur Veranschaulichung verschiedener Abhängigkeiten des Maximalstroms und der Maximalleistung vom gemessenen Widerstand.

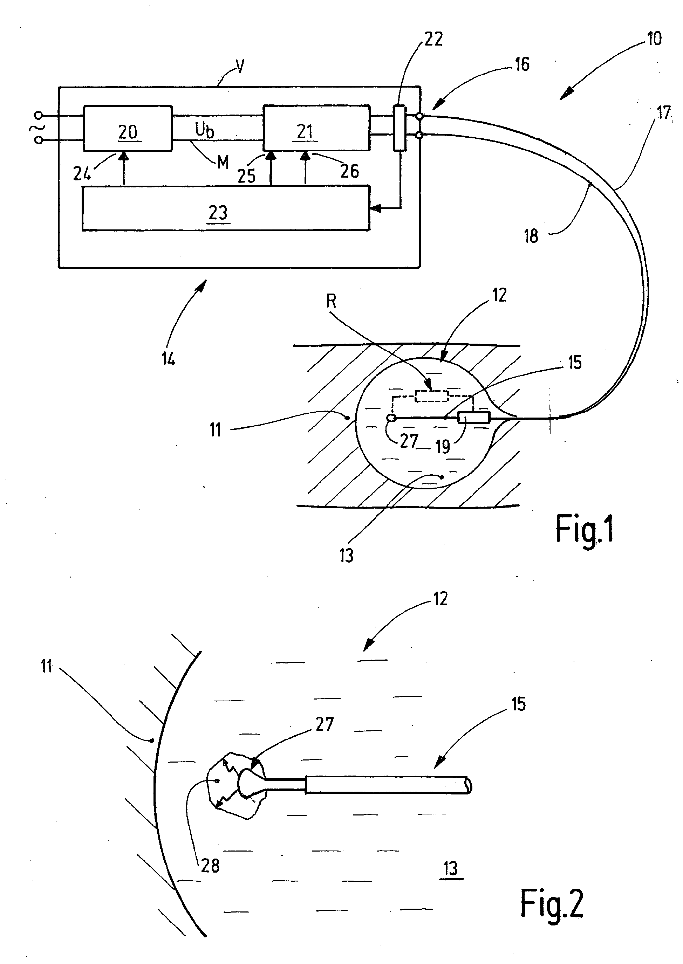

In Figur 1 ist eine Einrichtung 10 zur Behandlung biologischen Gewebes 11 veranschaulicht, das ein Lumen 12 begrenzt. Das Lumen 12 kann der Innenraum eines Hohlorgans oder auch eine sonstige in dem Gewebe 11 geschaffene Kavität sein. Typischerweise ist das Lumen 12 mit einer wässrigen Flüssigkeit 13, wie beispielsweise NaCl-Lösung, teilweise oder ganz gefüllt.

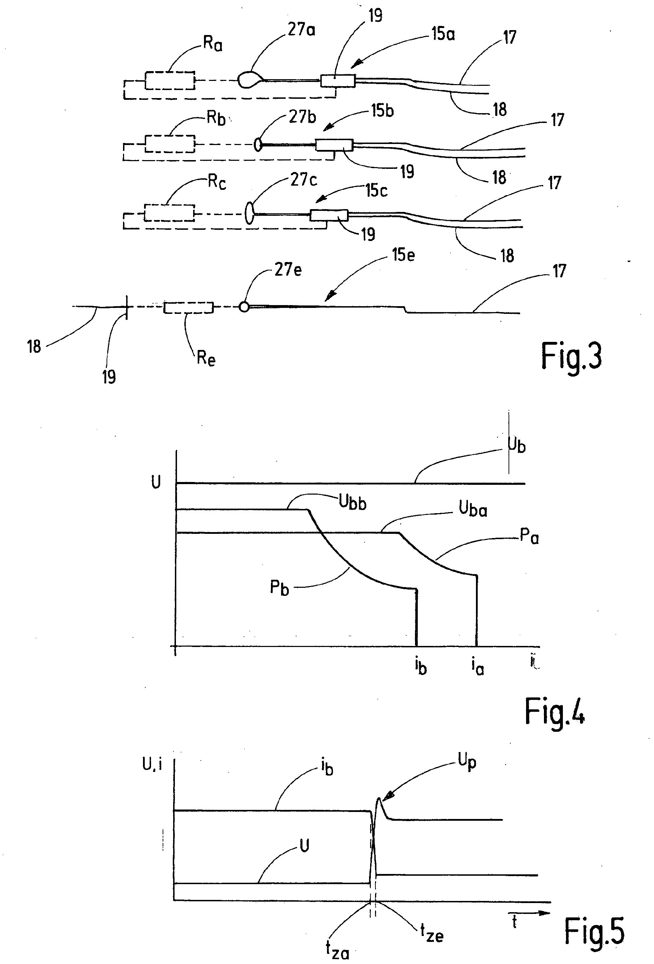

Zu der Einrichtung 10 gehört ein Gerät 14, das zur Speisung eines Instruments 15 mit elektrischem Strom dient. Dazu weist das Gerät 14 einen Ausgang 16 auf, an den das Instrument 15 angeschlossen oder anschließbar ist. Ist das Instrument 15, wie in Figur 1 veranschaulicht, ein bipolares Instrument sind beide Pole des Ausgangs 16 mit dem Instrument 15 verbunden. Ist das Instrument 15 hingegen monopolar ausgebildet(siehe Figur 3, unten), führt eine Leitung 17 von einem Pol des Ausgangs 16 zu dem Instrument 15, während eine andere Leitung 18 von einem anderen Pol des Ausgangs 16 zu einer Gegenelektrode 19 führt, die mit dem biologischen Gewebe 11 elektrisch verbunden ist. Die erste Leitung 17 ist eine HF-Leitung und die zweite Leitung 18 wird als Neutralleiter angesehen.

Das Gerät 14 enthält ein Netzteil 20, das mit einem elektrischen Versorgungsnetz verbindbar ist und zwischen zwei Leitungen V und M eine Betriebsspannung U_{b} bereitstellt. Diese dient zur Versorgung einer Baugruppe, die einen Generator 21 bildet. Der Generator 21 erzeugt aus der Betriebsspannung U_{b} eine hochfrequente Wechselspannung, die an dem Ausgang 16 abgegeben wird. Die Frequenz der Wechselspannung kann im Bereich von 100 kHz bis 10 MHz festgelegt sein.

Zur Erfassung des an das Instrument 15 abgegebenen Stroms und/oder zur Erfassung der an dem Ausgang 16 anliegenden Spannung kann beispielsweise zwischen dem Generator 21 und dem Ausgang 16 oder auch als Teil des Generators 21 eine Messeinrichtung 22 vorgesehen sein. Die Messeinrichtung 22 kann außer der an dem Ausgang 16 anliegenden Spannung und/oder dem durch den Ausgang 16 hinaus und hinein fließenden Strom bedarfsweise auch daraus abgeleitete Größen, wie zum Beispiel den an dem Ausgang 16 wirksamen ohmschen Widerstand und/oder die von dem Ausgang 16 abgegebene Wirkleistung, Scheinleistung und/oder Blindleistung bestimmen. Die erfassten und/oder bestimmten Größen (Spannung, Strom, Widerstand, Impedanz, Leistung usw.) werden an eine Steuereinrichtung 23 übergeben. Dabei kann die Messeinrichtung 22, nicht nur, wie in Figur 1 dargestellt, von der Steuereinrichtung 23 separat, sondern auch ganz oder teilweise als Teil derselben ausgebildet sein. Die Steuereinrichtung 23 und die Messeinrichtung 22 sind ebenso wie der Generator 21 und das Netzteil 20 als Funktionsblöcke zu verstehen, die sowohl auf gleichen als auch auf voneinander getrennten Hardwarebaugruppen aufgebaut sein können.

Die Steuereinrichtung 23 ist an einen Spannungsregeleingang 24 des Netzteils 20 angeschlossen. Über diesen Spannungsregeleingang 24 gibt die Steuereinrichtung 23 dem Netzteil 20 den Sollwert für die Betriebsspannung U_{b} vor. Eine in dem Netzteil 20 ausgebildete Regeleinrichtung regelt den Istwert der Betriebsspannung U_{b} dann auf den Sollwert aus, wobei es im Rahmen des Regelvorgangs zu temporären Abweichungen kommen kann. Dieser Regler kann auch in der Steuereinrichtung 23 realisiert sein.

Die Steuereinrichtung 23 ist außerdem mit einem Strombegrenzungseingang 25 verbunden, der an dem Generator 21 oder an dem Netzteil 20 oder auch an einer Strombegrenzungsbaugruppe ausgebildet sein kann. Die Strombegrenzungsbaugruppe kann zwischen dem Netzteil 20 und dem Generator 21 oder auch zwischen dem Generator 21 und dem Ausgang 16 angeordnet sein. Ein an den Strombegrenzungseingang 25 gegebenes Signal legt den an dem Ausgang 16 maximal abgebbaren Strom fest. Wird dieser erreicht, wird die Arbeit des Netzteils 20 und/oder des Generators 21 daran angepasst, den Stromgrenzwert nicht zu überschreiten.

Weiter ist ein Leistungsbegrenzungseingang 26 vorgesehen, der an dem Generator 21, an dem Netzteil 20 oder an einer zwischen dem Generator 21 und dem Netzteil 20 angeordneten Baugruppe vorgesehen sein kann. Das an dem Leistungsbegrenzungseingang 26 anstehende Signal legt die Maximalleistung fest, die an dem Ausgang 16 abgebbar ist.

Der Spannungsbegrenzungseingang 24, der Strombegrenzungseingang 25 und der Leistungsbegrenzungseingang 26 sind als Informationskanäle zu verstehen, die in jeder beliebigen elektrotechnischen und datenübertragungsmäßigen Art realisiert sein könne. Auch ist es möglich, auf einen gesonderten Leistungsbegrenzungseingang zu verzichten und die Leistungsbegrenzung durch Abstimmung der Signale an dem Spannungsbegrenzungseingang 24 und dem Strombegrenzungseingang 25 aufeinander zu erreichen.

Figur 2 dient der Veranschaulichung des Instruments 15 und seines Einsatzes in dem Lumen 12. Das Instrument 15 weist eine zum Beispiel in Form einer Schlinge oder eines Bügels ausgebildete Elektrode 27 auf, die mit der Leitung 17 verbunden ist. Die Elektrode 27 ist im ungezündeten Zustand nass und in vollflächiger Berührung mit der Flüssigkeit 13, d.h. sie steht somit in elektrischem Kontakt mit dieser. Zwischen der Elektrode 27 und der Gegenelektrode 19 ist deswegen ein in Figur 1 gestrichelt dargestellte Widerstand R messbar, der im Wesentlichen von der Größe und Form der Elektrode 27 bestimmt wird. Der Widerstand R setzt sich durch eine Zusammenschaltung des Widerstands zwischen der Elektrode 27 und der umgebenden Flüssigkeit 13 sowie dem Widerstand des Strompfads durch die Flüssigkeit 13 bis zu der Gegenelektrode 19 zusammen. Befindet sich die Elektrode 27 in Wandnähe, gibt es noch einen parallelen Strompfad durch das Gewebe. Ist die Gegenelektrode 19 nicht an dem Instrument 15 ausgebildet, sondern gesondert an dem Patienten anzubringen, setzt sich der elektrische Widerstand nur aus der Reihenschaltung des Übergangswiderstands zwischen der Elektrode 27 und der umgebenden Flüssigkeit 13 sowie dem Widerstand des Strompfads durch die Flüssigkeit 13 und dem Widerstand durch das Körpergewebe bis zu der Gegenelektrode 19 zusammen.

Figur 3 veranschaulicht symbolisch verschiedene Instrumente 15a, 15b, 15c, 15e und jeweils gestrichelt den Widerstand R zur entsprechenden Neutralelektrode. Während die Instrumente 15a bis 15c als monopolare Instrumente ausgebildet sind, die nur über die Leitung 17 versorgt werden, ist bei dem bipolaren Instrument 15e die als Gegenelektrode bezeichnete Gegenelektrode 19 direkt an dem Instrument 15e ausgebildet, das dann mit beiden Leitungen 17, 18 verbunden ist. Im Weiteren können sich die Instrumente 15a bis 15e durch Größe und Form ihrer jeweiligen Elektrode 27a, 27b, 27c, 27e unterscheiden. Z.B. können die Elektroden 27a, 27b, 27c, 27e als größere oder kleinere Drahtschlingen, Bandschlingen, Pilzförmige oder anderweitige Flächenelektroden ausgebildet sein.

Die Instrumente 15a bis 15e weisen, wenn sie mit der Flüssigkeit 13 in Berührung kommen und an das Gerät 14 angeschlossen sind, einen charakteristischen, zwischen der Elektrode 27 und der Gegenelektrode 19 zu messenden Widerstand Rₐ, R_{b}, R_{c} oder Rₑ auf und der von der Messeinrichtung 22 erfasst werden kann. Die Erfassung kann zum Beispiel unmittelbar bevor an der Elektrode 27 ein Plasma erzeugt werden soll oder auch ganz zu Beginn eines solchen Zündversuchs erfolgen, solange die Flüssigkeit 13 noch ungestört an der Elektrode 27 anliegt. Es ist auch möglich, einen Messzyklus durchzuführen, bevor ein Zündversuch unternommen wird.

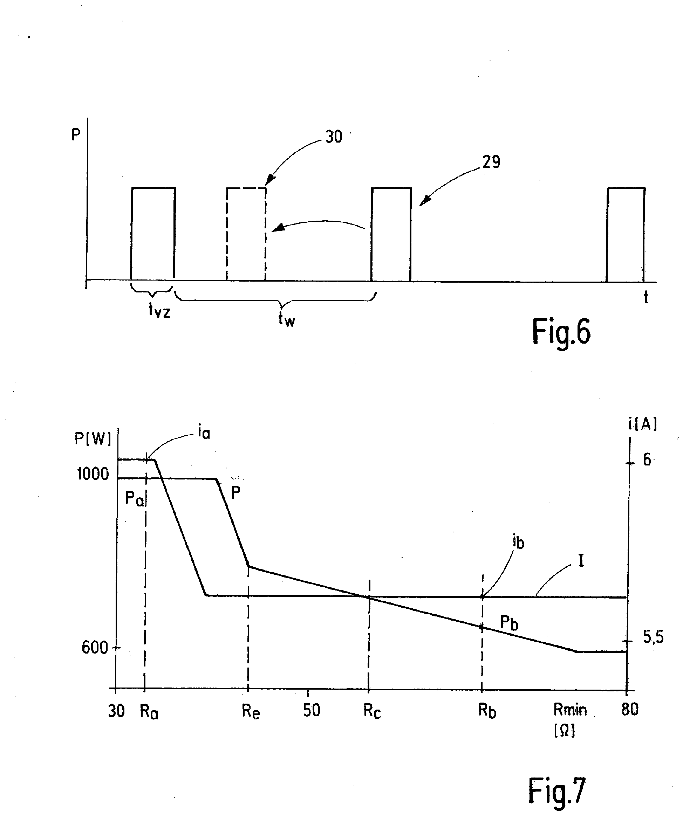

Zur Widerstandsbestimmung wird an dem Ausgang 16 eine Spannung bereitgestellt. Diese kann mit der Spannung zum Zünden eines Plasmas übereinstimmen oder auch niedriger als diese sein. Der an dem Ausgang 16 fließende Strom kann dann von der Messeinrichtung 22 erfasst werden. Aus der gemessenen Spannung und dem gemessenen Strom lässt sich die Impedanz und/oder der Widerstand Rₘᵢₙ bestimmen. Dieser hat vor Durchführung eines ersten Zündversuchs einen Wert Rₐ, R_{b}, R_{c}, Rₑ der jeweils als charakteristisch für das jeweilige Instrument 15a bis 15e angesehen werden kann. In diesem Zusammenhang wird auf Figur 7 verwiesen. Diese veranschaulicht auf ihrer horizontalen Achse verschiedene Werte Rₘᵢₙ nämlich Rₐ, Rₑ, R_{c}, R_{b} für die verschiedenen Instrumente 15a, 15e, 15c und 15b. Typischerweise liegen diese Werte zwischen 20 Ohm und 100 Ohm, wobei andere Werte nicht ausgeschlossen sind.

Figur 7 dient im Weiteren zur Veranschaulichung einer Zündsteuerstrategie, die von der Steuereinrichtung 23 umgesetzt wird. Dazu wird außerdem und ergänzend auf die Figuren 4 und 5 Bezug genommen:

Zur Zündung eines Plasmas 28 innerhalb der Flüssigkeit 13 (siehe Figur 2) oder an der Wand des Lumens 12 stellt das Gerät 14 hochfrequente Wechselspannung bereit, die einen Stromfluss durch die Flüssigkeit 13 und, falls die Gegenelektrode 19 nicht an dem Instrument 15 selbst, sondern an dem Gewebe 11 angebracht ist, durch das Gewebe zur Folge hat. Infolge des direkten nassen Kontakts der Flüssigkeit 13 zu der Elektrode 27 ist der vorhandene Widerstand sehr klein und es fließt ein maximaler Strom. Die Steuereinrichtung 23 begrenzt dabei im Netzteil 20 oder im Generator 21 den Strom auf einen zu dem jeweiligen Instrument 15a, 15b, 15c oder 15e jeweils passenden Wert. Dieser Wert hängt von dem Widerstand Rₐ bis Rₑ ab, den die Messeinrichtung 22 gerade jetzt oder zu einem dem Zündversuch vorausgehenden Zeitpunkt bestimmt hat.

Figur 7 veranschaulicht anhand der Kurve I verschiedene Maximalstromwerte für die verschiedenen Instrumente 15a, 15b, 15c oder 15e mit den verschiedenen Widerständen Rₐ bis Rₑ. Entsprechend wird die Generatorausgangscharakteristik zum Beispiel für das Instrument 15b auf den Wert i_{b} in Figur 4 festgelegt. Ebenso wird gemäß Figur 7 die maximale Leistung p_{b} für dieses Instrument 15b festgelegt. Damit wird, sobald die Strombegrenzung nicht aktiv ist, weil eine Dampfblasenbildung an der Elektrode 27 beginnt und eine Stromverminderung verursacht, die Leistung gemäß dem Kurvenabschnitt p_{b} begrenzt. Während des gesamten Zündvorgangs wird der Generator 21 mit der Betriebsspannung U_{bb} versorgt, die vorzugsweise niedriger festgelegt ist als die Betriebsspannung U_{b}, die für den Generator bei gezündeten Plasma 278 bereitgestellt wird. Im niederohmigen Zustand befindet sich das System in der Strom- oder Leistungsbegrenzung, mit Entstehen der Dampfblase hingegen in der Spannungsbegrenzung.

Die Reduktion der Betriebsspannung U_{b} auf den Wert U_{bb}, die Leistungsbegrenzung p_{b} und die Strombegrenzung i_{b} nach dem Diagramm in Figur 4 fördern einen instrumentengerechten Zündvorgang mit geringer Funkenbildung und stabilen Plasmaaufbau. Bislang hinzunehmende schädliche Effekte werden vermieden. Dies ist auch aus dem vereinfachten Diagramm nach Figur 5 ersichtlich, das sich insbesondere mit dem Zünden des Plasmas 28 befasst. Das Diagramm veranschaulicht in seiner linken Hälfte zunächst bei an die Elektrode 27 angelegter Spannung U einen hohen Strom i_{b}, der durch die Strombegrenzung (i_{b} in Figur 4 senkrechter Zweig der Kurve) festgelegt ist. Wegen des niedrigen Widerstands von einigen wenigen 10 Ohm liegt lediglich eine geringe Spannung U an der Elektrode 27b an. Die Spannungsvorgabe in diesem Zustand ist aber höher als im Schnitt. Dies ist notwendig, da ansonsten eine zunächst auftretende Spannungsüberhöhung den Spannungsregler veranlassen würde, die Spannung zu weit herunter zu regeln, wodurch das Plasma erlöschen könnte.

Zu einem Zeitpunkt t_{za} bildet sich um die Elektrode 27 eine Dampfblase, die zunächst elektrisch nicht leitet und deswegen den Stromfluss bis zum Spannungsdurchschlag unterbricht oder zumindest stark einschränkt. Die abrupte Stromunterbrechung oder Stromreduktion führt typischerweise zu einer Spannungsspitze an dem Generator 21 oder dem Netzteil 20 insbesondere infolge der dort in Induktivitäten gespeicherten Energie. Diese Spannungsspitze ist in Figur 5 durch Uₚ symbolisiert und führt typischerweise zu einer störenden Licht- und gegebenenfalls Schallimpulserzeugung, die auch als "Funkenspiel" bezeichnet wird. Durch die Reduktion der Betriebsspannung auf den Wert U_{bb} (z.B. 550 V) und durch die Beschränkung der Leistung auf den instrumentenspezifischen Wert p_{b} wird jedoch beim Durchlaufen der Kurve p_{b} gemäß der Kurve in Figur 4 das entstehende Funkenspiel auf ein Minimum reduziert. Die Spannung U steigt deswegen nach dem Zeitpunkt t_{za} bis zum Zeitpunkt t_{ze} mit einem geringen Überschwingen oder ohne Überschwinger an, während der Strom im Gegenzug auf seinen Betriebswert abfällt.

Wird hingegen ein anderes Instrument, beispielsweise das Instrument 15a, eingesetzt und weist dieses in der Berührung mit der Flüssigkeit 13 einen anderen, geringeren Widerstand Rₐ (siehe Figur 7) auf, stellt die Steuereinrichtung 23 für den Zündvorgang gemäß Figur 4 einen höheren Maximalstrom iₐ und eine höhere Maximalleistung pₐ sowie eine wiederum passende Betriebsspannung U_{ba} auf, die größer oder kleiner als U_{bb} sein oder auch mit dieser übereinstimmen kann. Wiederum ergibt sich ein Zündvorgang mit minimalem Funkenspiel und ruhigem Übergang zum stabilen Plasmaerhalt.

Die Steuereinrichtung 23 ist weiter darauf eingerichtet, einen Zündversuch nach Ablauf einer Zündversuchszeit t_{VZ} abzubrechen, falls keine Zündung festgestellt worden ist. Wie das Diagramm in Figur 6 erkennen lässt, wird danach eine Wartephase t_{W} durchlaufen, innerhalb derer kein weiterer Zündversuch unternommen wird. Typischerweise beträgt die Wartezeit t_{W} mehrere 100 ms, zum Beispiel 0,8 s. Damit wird der Leistungseintrag in die Flüssigkeit 13 und/oder das Gewebe 11 begrenzt.

Zur Erhöhung des Komforts kann vorgesehen werden, dass die Steuereinrichtung 23 die Wartezeit t_{W} unter bestimmten Bedingungen verkürzt bzw. nicht einhält. Dazu kann die Steuereinrichtung 23 darauf eingerichtet sein, die zwischen der Elektrode 27 und der Gegenelektrode 19 verrichtete elektrische Arbeit für einen bestimmten Erfassungszeitraum zu erfassen. Beispielsweise kann dieser Erfassungszeitraum 1 s und die darin maximal zu verrichtende Arbeit 400 Ws betragen. Nach Ablauf eines solchen Erfassungsintervalls kann die Erfassung neu gestartet werden. Liegt nun beispielsweise der erste Zündversuch (in Figur 6 ganz links) in einem Überwachungsintervall, in dem außer dem ersten Zündversuch in der Zündversuchszeit t_{VZ} noch kein weiterer Zündversuch stattgefunden hat und ist die maximale elektrische Arbeit in diesem Überwachungsintervall noch nicht erreicht, kann die Wartezeit t_{W} verkürzt und der sonst erst nach der Wartezeit zu unternehmende zweite Zündversuch, wie in Figur 6 durch einen Pfeil angedeutet, als Zündversuch 30 vorzeitig ausgeführt werden.

Es ist auch möglich, die Verkürzung bzw. den vorzeitigen Abbruch des Wartezeitintervalls t_{W} dann auszulösen, wenn eine Annäherung der Elektrode 27 an das biologische Gewebe 11 festgestellt wird. Dies geht typischerweise mit einer Veränderung, z.B. Erhöhung des elektrischen Widerstands zwischen der Elektrode 27 und der Gegenelektrode 19 einher. Eine solche Widerstandsveränderung kann als auslösendes Ereignis für den Abbruch des Wartezeitintervalls, d.h. zum Unternehmen eines vorgezogenen Zündversuchs 30 (Figur 6) genutzt werden. Bedarfsweise können mehrere Zündversuche in kürzerer Folge unternommen werden. Dies kann in einer Ausführungsvariante unter der Bedingung stehen, dass in einem festgelegten Überwachungszeitraum eine festgelegte Grenze für die verrichtete elektrische Arbeit noch nicht erreicht oder überschritten ist.

Ein Gerät zur Speisung eines medizinischen Instruments 15 zur Behandlung von biologischem Gewebe 11 durch Einwirkung eines Plasmas 28 ist erfindungsgemäß in besonderer Weise auf das Zünden und den stabilen Aufbau eines Plasmas 28 an der Elektrode 27 des Instruments 15 eingerichtet. Dazu weist das Gerät 14 eine Steuereinrichtung 23 auf, die während eines Zündversuchs vorzugsweise instrumentenspezifisch den an das Instrument 15 zu liefernden Strom begrenzt und/oder die an das Instrument 15 abzugebende elektrische Leistung begrenzt und/oder dabei mit reduzierter Betriebsspannung U_{bb} arbeitet. Mit dieser Maßnahme wird für eine große Vielfalt anschließbarer Instrumente 15a - 15e jeweils ein schneller stabiler Plasmaaufbau mit geringem Funkenspiel erreicht.

### Bezugszeichen:

- 10: Einrichtung
- 11: biologisches Gewebe
- 12: Lumen
- 13: Flüssigkeit
- 14: Gerät
- 15: Instrument (in allgemeiner Bezugnahme)
- 15a - 15e: Instrument (in spezifischer Bezugnahme)
- 16: Ausgang
- 17: erste Leitung
- 18: zweite Leitung (Neutralleitung)
- 19: Gegenelektrode
- 20: Netzteil
- V, M: Leitungen
- U_{b}: Betriebsspannung (z.B. 600 Vₚ)
- 21: Generator
- 22: Messeinrichtung
- 23: Steuereinrichtung
- 24: Spannungsregeleingang
- 25: Strombegrenzungseingang
- 26: Leistungsbegrenzungseingang
- 27: Elektrode (in allgemeiner Bezugnahme)
- 27a - 27e: Elekrtode (in spezifischer Bezugnahme)
- R: Widerstand
- Rₘᵢₙ: minimaler Messwert des Widerstands R
- Rₐ-Rₑ: instrumentspezifischer minimaler Widerstand Rₘᵢₙ
- 28: Plasma
- I: instrumentenabhängige Maximalstromkurve
- P: instrumentenabhängige Maximalleistungskurve
- i_{b}: Maximalstrom für Instrument 15b
- iₐ: Maximalstrom für Instrument 15a
- p_{b}: Maximalleistung für Instrument 15b (
- pₐ: Maximalleistung für Instrument 15a
- U_{bb}: Betriebsspannung für Instrument 15b)
- U_{ba}: Betriebsspannung für Instrument 15a
- t_{VZ}: Zündversuchszeit (50ms-500ms,)
- t_{W}: Wartezeitintervall (0,5s-1s,)
- 29: späterer Zündversuch
- 30: zeitlich vorgezogener Zündversuch

## Patentansprüche

1. Gerät(14) zur Speisung eines medizinischen Instruments (15) mit einer Elektrode (27) zur Behandlung von mit einer wässrigen Flüssigkeit (13) in Kontakt stehendem biologischem (11) Gewebe durch Einwirkung eines Plasmas (28),
wobei das Gerät (14) aufweist:
einen Generator (21) zur Abgabe einer hochfrequenten Wechselspannung, wobei der Generator (21) mit einem Ausgang (16) verbunden ist, an den das Instrument (15) anschließbar ist,
ein Netzteil (20), das mit dem Generator (21) verbunden ist, um diesen mit einer Betriebsspannung (U_{b}) zu versorgen,
eine Steuereinrichtung (23) zur Steuerung des Generators (21) und/oder des Netzteils (20),
mit einer Messeinrichtung (22) zur Bestimmung des elektrischen Widerstands (Rₘᵢₙ) des an den Ausgang (22) angeschlossenen Instruments (15),
wobei die Steuereinrichtung (23) dazu eingerichtet ist, wenigstens für eine Zündversuchszeit (t_{ZV}) die Betriebsspannung (U_{b}) und/oder die an das Instrument (15) abgebbare Leistung (P) und/oder den an das Instrument (15) lieferbaren Maximalstrom (i) entsprechend des gemessenen Widerstands (Rₘᵢₙ) vorzugeben und das Netzteil (20) und/oder den Generator (21) entsprechend zu steuern,
**dadurch gekennzeichnet, dass** die Steuereinrichtung (23) dazu eingerichtet ist, jeweils anhand des gemessenen minimalen Widerstands (Rₘᵢₙ) an der in die Flüssigkeit (13) eingetauchten, aber nicht gezündeten Elektrode (27), eine Anpassung des Zündvorgangs an unterschiedlichen Instrumente (15) mit unterschiedlichen Elektrodenformen vorzunehmen.

2. Gerät nach Anspruch 1, **dadurch gekennzeichnet, dass** das Netzteil (20) einen Spannungsregeleingang (24) aufweist, der mit der Steuereinrichtung (23) verbunden ist.

3. Gerät nach Anspruch einem der vorstehenden Ansprüche, **dadurch gekennzeichnet, dass** das Netzteil (20) und/oder der Generator (21) einen Strombegrenzungseingang (25) aufweist, der mit der Steuereinrichtung (23) verbunden ist.

4. Gerät nach Anspruch einem der vorstehenden Ansprüche, **dadurch gekennzeichnet, dass** das Netzteil (20) und/oder der Generator (21) einen Leistungsbegrenzungseingang (26) aufweist, der mit der Steuereinrichtung (23) verbunden ist.

5. Gerät nach einem der vorstehenden Ansprüche, **dadurch gekennzeichnet, dass** die Steuereinrichtung (23) eine Betriebsstatuserkennungseinrichtung aufweist, die dazu eingerichtet ist, den Beginn eines Zündversuchs zu erkennen, während dessen die von dem Generator (21) an das Instrument (15) gelieferte Wechselspannung an dem Instrument (15) ein Plasma (28) aufbaut.

6. Gerät nach einem der vorstehenden Ansprüche, **dadurch gekennzeichnet, dass** die Steuereinrichtung (23) dazu eingerichtet ist, während eines Zündversuchs einen Sollwert für die Betriebsspannung (U_{b}) vorzugeben, der höher ist, als während des Betriebs nach dem Zündversuch.

7. Gerät nach Anspruch einem der vorstehenden Ansprüche, **dadurch gekennzeichnet, dass** die Steuereinrichtung (23) eine Betriebsstatuserkennungseinrichtung aufweist, die dazu eingerichtet ist, das Ende eines Zündversuchs oder das Verlöschen des Plasmas zu erkennen, während dessen die von dem Generator (21) an das Instrument (15) gelieferte Wechselspannung an dem Instrument (15) zum Aufbau eines Plasmas (28) geführt hat.

8. Gerät nach Anspruch einem der vorstehenden Ansprüche, **dadurch gekennzeichnet, dass** die Steuereinrichtung (23) eine Betriebsstatuserkennungseinrichtung aufweist, die dazu eingerichtet ist, das Ende eines Zündversuchs zu erkennen, während dessen die von dem Generator (21) an das Instrument (15) gelieferte Wechselspannung an dem Instrument (15) nicht zum Aufbau eines Plasma (28) aufbaut.

9. Gerät nach einem der vorstehenden Ansprüche, **dadurch gekennzeichnet, dass** die Steuereinrichtung (23) dazu eingerichtet ist, nach einem fehlgeschlagenen Zündversuch in einer Wartephase (t_{W}) eine vorgegebene Pausenzeit einzuhalten und dann einen erneuten Zündversuch zu starten.

10. Gerät nach Anspruch 9, **dadurch gekennzeichnet, dass** die Steuereinrichtung (23) dazu eingerichtet ist, die an das Instrument (15) gelieferte elektrische Arbeit (W) zu erfassen.

11. Gerät nach Anspruch 10, dadurch die Steuereinrichtung (23) dazu eingerichtet ist, eine Annäherung eines in einem Lumen (12) platzierten Instruments (15) an biologisches Gewebe (11) zu erfassen und, sofern sich die Steuereinrichtung (23) in der Wartephase befindet und der Impedanzanstieg einen Grenzwert übersteigt die Wartephase abzubrechen und einen erneuten Zündversuch zu starten.

12. Gerät nach einem der vorstehenden Ansprüche, **dadurch gekennzeichnet, dass** die Steuereinrichtung (23) dazu eingerichtet ist, Betriebsspannung (U_{b}) und/oder die an das Instrument (15) abgebbare Leistung (P) und/oder den an das Instrument (15) lieferbaren Maximalstrom (i) in einer sich an die Zündphase anschließenden Betriebsphase entsprechend des vor Zündung des Plasmas (28) gemessenen Widerstands (Rₘᵢₙ) vorzugeben und das Netzteil (20) und/oder den Generator (21) entsprechend zu steuern.

13. Verfahren zum Vorgeben von Betriebseinstellungen zum Zünden eines Plasmas (28) in wässriger Umgebung an einer Elektrode (27) eines medizinischen Instruments (15), die mit einer wässrigen Flüssigkeit (13) in Berührung steht,
wobei die Elektrode (15) mit einem einen Gerät (14) verbunden ist, das zur Abgabe einer hochfrequenten Spannung eingerichtet ist,
wobei vor der Beaufschlagung der Elektrode (27) mit der hochfrequenten Spannung zur Zündung des Plasmas (28) der elektrische Widerstand (R) zwischen der Elektrode und der wässrigen Umgebung ermittelt und anhand dieses Widerstands (R) Betriebseinstellungen des Geräts (14) vorgenommen werden, wobei die Betriebseinstellungen eine Leerlaufspannung (U) und/oder eine an das Instrument (15) abgebbare Leistung (P) und/oder einen an das Instrument (15) lieferbaren Maximalstrom (i) betreffen, **gekennzeichnet dadurch, dass** die Steuereinrichtung (23) dazu eingerichtet ist, jeweils anhand des gemessenen minimalen Widerstands (Rₘᵢₙ) an der in die Flüssigkeit (13) eingetauchten, aber nicht gezündeten Elektrode (27), eine Anpassung des Zündvorgangs an unterschiedlichen Instrumente (15) mit unterschiedlichen Elektrodenformen vorzunehmen.

14. Verfahren nach Anspruch 13, **dadurch gekennzeichnet, dass** die Betriebseinstellungen vorzugsweise nur eine Zündphase betreffen, innerhalb derer an der Elektrode (27) anliegendes Wasser (13) verdampft und in der entstehenden Dampfblase ein Plasma (28) gezündet wird.

## Claims

1. An apparatus (14) for supplying a medical instrument (15) having an electrode (27) for the treatment of biological tissue (11) in contact with an aqueous liquid (13) due to the action of a plasma (28), wherein the apparatus (14) comprises a generator (21) for the output of a high-frequency ac voltage, in which case the generator (21) is connected to an outlet (16) that can be connected to the instrument (15),
a power supply (20) that is connected to the generator (21) in order to supply said power supply with an operating voltage (U_{b}),
a control device (23) for controlling the generator (21) and/or the power supply (20), with a measuring device (22) for determining the electrical resistance (Rₘᵢₙ) of the instrument (15) connected to the output (22),
wherein the control device (23) is configured to specify, at least for a time period (t_{ZV}), the operating voltage (U_{b}) and/or the power (P) to be output to the instrument (15) and/or the maximum current (i) deliverable to the instrument (15) consistent with the measured resistance (Rₘᵢₙ) and to control the power supply (20) and/or the generator (21) accordingly, **characterized in that** the control device (23) is set up to adapt the ignition process to different instruments (15) with different electrode shapes on the basis of the measured minimum resistance (Rₘᵢₙ) at the electrode (27) immersed in the liquid (13) but not ignited in each case.

2. The apparatus according to Claim 1, **characterized in that** the power supply (20) has a voltage regulation inlet (24) that is connected to the control device (23).

3. The apparatus according to one of the previous claims, **characterized in that** the power supply (20) and/or the generator (21) has a current limitation inlet (25) that is connected to the control device (23).

4. The apparatus according to one of the previous claims, **characterized in that** the power supply (20) and/or the generator (21) has a power limitation inlet (26) that is connected to the control device (23).

5. The apparatus according to one of the previous claims, **characterized in that** the control device (23) comprises an operating status detection arrangement that is configured to detect the start of an ignition test during which the ac voltage supplied by the generator (21) to the instrument (15) develops a plasma (28) on the instrument (15).

6. The apparatus according to one of the previous claims, **characterized in that** the control device (23) is configured to specify, during the ignition test, a nominal value for the operating voltage (U_{b}), said value being greater than during operation after the ignition test.

7. The apparatus according to one of the previous claims, **characterized in that** the control device (23) comprises an operating status detection arrangement that is configured to detect the end of an ignition test or the extinguishing of the plasma, during which the ac voltage supplied to the instrument (15) by the generator (21) has resulted in the development of a plasma (28) on the instrument (15).

8. The apparatus according to one of the previous claims, **characterized in that** the control device (23) comprises an operating status detection arrangement that is configured to detect the end of an ignition test, during which the ac voltage supplied to the instrument (15) by the generator (21) does not result in the development of a plasma (28) on the instrument (15) .

9. The apparatus according to one of the previous claims, **characterized in that** the control device (23) is configured - after a failed ignition test - to maintain a specified pause interval during a wait phase (t_{W}) and then to start another ignition test.

10. The apparatus according to Claim 9, **characterized in that** the control device (23) is configured to detect the electrical work (W) supplied to the instrument (15).

11. The apparatus according to Claim 10, **characterized in that** the control device (23) is configured to detect an approach of an instrument (15) placed in a lumen (12) toward the biological tissue (11) and to interrupt the waiting period and start another ignition test, provided the control device (23) is in the waiting period.

12. The apparatus according to one of the previous claims, **characterized in that** the control device (23) is configured to specify the operating voltage (U_{b}) and/or the power (P) deliverable to the instrument (15) and/or the maximum current (15) deliverable to the instrument (15) during an operating phase following the ignition phase, consistent with the resistance (Rₘᵢₙ) measured before the ignition of the plasma (28), and to control the power supply (20) and/or the generator (21) accordingly.

13. A method for specifying operating settings for igniting a plasma (28) in an aqueous environment on an electrode (27) of a medical instrument (15) that is in contact with an aqueous fluid (13),
wherein the electrode (15) is connected to an apparatus (14) that is configured for the output of a high-frequency voltage,
wherein, before the high-frequency voltage is applied to the electrode (27) for igniting the plasma (28), the electrical resistance (R) between the electrode and the aqueous environment is determined and, considering this resistance (R), operating settings of the apparatus (14) are performed,
wherein the operating settings relate to an no-load voltage (U) and/or a power (P) which can be output to the instrument (15) and/or a maximum current (i) which can be supplied to the instrument (15), **characterized in that** the control device (23) is configured to adapt the ignition process to different instruments (15) with different electrode shapes on the basis of the measured minimum resistance (Rₘᵢₙ) at the electrode (27) immersed in the liquid (13) but not ignited in each case.

14. The method according to Claim 13, **characterized in that** the operating settings preferably involve only one ignition phase, during which water (13) present on the electrode (27) is evaporated and a plasma (28) is ignited in the developing vapor bubble.

## Revendications

1. Appareil (14) destiné à l'alimentation d'un instrument médical (15) doté d'une électrode (27) pour le traitement de tissu biologique (11) en contact avec un liquide aqueux (13), par action d'un plasma (28),
l'appareil (14) présentant :
un générateur (21) destiné à délivrer une tension alternative à haute fréquence, le générateur (21) étant relié à une sortie (16) à laquelle peut être raccordé l'instrument (15),
un bloc d'alimentation (20) qui est relié au générateur (21) afin d'alimenter celui-ci avec une tension de fonctionnement (U_{b}),
un dispositif de commande (23) destiné à commander le générateur (21) et/ou le bloc d'alimentation (20),
comprenant un dispositif de mesure (22) destiné à déterminer la résistance électrique (Rₘᵢₙ) de l'instrument (15) raccordé à la sortie (22),
le dispositif de commande (23) étant conçu pour prédéfinir, au moins pendant un temps d'essai d'amorçage (t_{zv}), la tension de fonctionnement (U_{b}) et/ou la puissance (P) pouvant être délivrée à l'instrument (15) et/ou le courant maximal (i) pouvant être fourni à l'instrument (15), en fonction de la résistance (Rₘᵢₙ) mesurée, et pour commander de manière correspondante le bloc d'alimentation (20) et/ou le générateur (21),
**caractérisé en ce que**
le dispositif de commande (23) est conçu pour procéder, respectivement à l'aide de la résistance minimale (Rₘᵢₙ) mesurée sur l'électrode (27) plongée dans le liquide (13) mais non amorcée, à une adaptation de l'opération d'amorçage à des instruments (15) différents dotés de formes d'électrode différentes.

2. Appareil selon la revendication 1, **caractérisé en ce que** le bloc d'alimentation (20) présente une entrée de réglage de tension (24) qui est reliée au dispositif de commande (23).

3. Appareil selon la revendication l'une des revendications précédentes, **caractérisé en ce que** le bloc d'alimentation (20) et/ou le générateur (21) présente(nt) une entrée de limitation de courant (25) qui est reliée au dispositif de commande (23).

4. Appareil selon la revendication l'une des revendications précédentes, **caractérisé en ce que** le bloc d'alimentation (20) et/ou le générateur (21) présente(nt) une entrée de limitation de puissance (26) qui est reliée au dispositif de commande (23).

5. Appareil selon l'une des revendications précédentes, **caractérisé en ce que** le dispositif de commande (23) présente un dispositif de détection d'état de fonctionnement qui est conçu pour détecter le début d'une tentative d'amorçage, pendant laquelle la tension alternative fournie à l'instrument (15) par le générateur (21) crée un plasma (28) sur l'instrument (15).

6. Appareil selon l'une des revendications précédentes, **caractérisé en ce que** le dispositif de commande (23) est conçu pour prédéfinir, au cours d'une tentative d'amorçage, une valeur de consigne pour la tension de fonctionnement (U_{b}) qui est plus élevée que pendant le fonctionnement après la tentative d'amorçage.

7. Appareil selon la revendication l'une des revendications précédentes, **caractérisé en ce que** le dispositif de commande (23) présente un dispositif de détection d'état de fonctionnement qui est conçu pour détecter la fin d'une tentative d'amorçage ou l'extinction du plasma, pendant laquelle la tension alternative fournie à l'instrument (15) par le générateur (21) a conduit à la création d'un plasma (28) sur l'instrument (15).

8. Appareil selon la revendication l'une des revendications précédentes, **caractérisé en ce que** le dispositif de commande (23) présente un dispositif de détection d'état de fonctionnement qui est conçu pour détecter la fin d'une tentative d'amorçage, pendant laquelle la tension alternative fournie à l'instrument (15) par le générateur (21) n'établit pas la création d'un plasma (28) sur l'instrument (15).

9. Appareil selon l'une des revendications précédentes, **caractérisé en ce que** le dispositif de commande (23) est conçu pour respecter, après une tentative d'amorçage infructueuse, une durée de pause prédéfinie dans une phase d'attente (t_{w}) et de lancer ensuite une nouvelle tentative d'amorçage.

10. Appareil selon la revendication 9, **caractérisé en ce que** le dispositif de commande (23) est conçu pour détecter le travail électrique (W) fourni à l'instrument (15).

11. Appareil selon la revendication 10, **caractérisé en ce que** le dispositif de commande (23) est conçu pour détecter lorsqu'un instrument (15) placé dans une lumière (12) approche de tissus biologiques (11), et, dans la mesure où le dispositif de commande (23) se trouve dans la phase d'attente et l'augmentation de l'impédance dépasse une valeur limite, pour interrompre la phase d'attente et lancer une nouvelle tentative d'amorçage.

12. Appareil selon l'une des revendications précédentes, **caractérisé en ce que** le dispositif de commande (23) est conçu pour prédéfinir la tension de fonctionnement (U_{b}) et/ou la puissance (P) pouvant être délivrée à l'instrument (15) et/ou le courant maximal (i) pouvant être fourni à l'instrument (15), en fonction de la résistance (Rₘᵢₙ) mesurée avant l'amorçage du plasma (28), au cours d'une phase de fonctionnement succédant à la phase d'amorçage, et pour commander de manière correspondante le bloc d'alimentation (20) et/ou le générateur (21).

13. Procédé pour prédéfinir des réglages de fonctionnement en vue de l'amorçage d'un plasma (28) dans un environnement aqueux sur une électrode (27) d'un instrument médical (15) qui est en contact avec un liquide aqueux,
l'électrode (15) étant reliée à un appareil (14) qui est conçu pour délivrer une tension à haute fréquence,
sachant qu'avant l'application de la tension à haute fréquence à l'électrode (27) en vue de l'amorçage du plasma (28), la résistance électrique (R) entre l'électrode et l'environnement aqueux est déterminée, et des réglages de fonctionnement de l'appareil (14) sont réalisés sur la base de cette résistance (R), les réglages de fonctionnement concernant une tension à vide (U) et/ou une puissance (P) pouvant être délivrée à l'instrument (15) et/ou un courant maximal (i) pouvant être fourni à l'instrument (15), **caractérisé en ce que** le dispositif de commande (23) est conçu pour procéder à une adaptation de l'opération d'amorçage à des instruments (15) différents avec des formes d'électrodes différentes, respectivement sur la base de la résistance minimale (Rₘᵢₙ) mesurée sur l'électrode (27) plongée dans le liquide (13) mais non amorcée.

14. Procédé selon la revendication 13, **caractérisé en ce que** les réglages de fonctionnement concernent de préférence une seule phase d'amorçage au cours de laquelle l'eau (13) appliquée contre l'électrode (27) s'évapore, et un plasma (28) est amorcé dans la bulle de vapeur créée.
